# EUROPÄISCHE PATENTSCHRIFT

(11) **EP 0 632 078 B1**
(45) Veröffentlichungstag und Bekanntmachung des Hinweises auf die Patenterteilung: **26.02.2003**
(21) Anmeldenummer: 94810375.9
(22) Anmeldetag: 23.06.1994
(51) Int. Cl.: C08G 59/14, C08G 59/42, C08G 59/04, C08F 290/14

(54) **Epoxyacrylate**
Epoxyacrylates
Résines époxyacrylates

(30) Priorität: 02.07.1993 CH 200393
(43) Veröffentlichungstag der Anmeldung: 04.01.1995
(60) Teilanmeldung: 02028222.4
(73) Patentinhaber: Vantico AG, 4057 Basel (CH)
(72) Erfinder: Roth, Martin, Dr., CH-1735 Giffers (CH); Salvin, Roger, Dr., D-79576 Weil am Rhein (DE); Meier, Kurt, Dr., CH-4106 Therwil (CH); Sailer, Bernhard, Dr., CH-4058 Basel (CH); Wiesendanger, Rolf, Dr., CH-4125 Riehen (CH)
(74) Vertreter: Dannappel, Hans-Jochen, Dr.

(56) Entgegenhaltungen:
- EP-A- 0 167 051
- EP-A- 0 292 219
- EP-A- 0 321 824
- WO-A-89/07785
- FR-A- 2 220 563
- FR-A- 2 320 961
- JP-A- 5 032 746
- US-A- 4 623 701
- DATABASE WPI Derwent Publications Ltd., London, GB; AN 89-232343(32) & JP-A-1 168 722 (ASAHI CHIBA KK) 4. Juli 1989

## Beschreibung

Die Erfindung betrifft neue höhermolekulare Epoxyacrylate sowie neue höhermolekulare carboxylgruppenhaltige Epoxyacrylate, Verfahren zu deren Herstellung, die Verwendung dieser Epoxyacrylate in Photoresistformulierungen und die Anwendung dieser Formulierungen vor allem auf dem Gebiet der Leiterplatten, z.B. als Lötstoppresist oder als Primärresist (Ätzresist oder Galvanoresist) und der Druckplatten.

Epoxyacrylate sind in grosser Zahl bekannt und werden unter anderem auch in Zusammensetzungen verwendet, die als Photoresistformulierungen dienen.

So beschreibt z.B. die EP 0,273,729 Zusammensetzungen für Lötstoppresists, die Umsetzungsprodukte aus Epoxy-novolakharzen mit Acrylsäure und cyclischen Carbonsäureanhydriden enthalten. Sie sind wässrig-alkalisch entwickelbar und weisen eine gute Wärmebeständigkeit und Photoempfindlichkeit auf. Allerdings lässt die Chemikalienbeständigkeit noch zu wünschen übrig.

Die EP 0,418,011 offenbart Zusammensetzungen für Lötstoppresists, ebenfalls basierend auf Reaktionsprodukten von Epoxy-kresolnovolaken mit Acrylsäure und cyclischen Dicarbonsäureanhydriden, wobei 0,4 bis 0,9 Aequivalente Acrylsäure pro Aequivalent Epoxygruppe eingesetzt werden, so dass das Endprodukt zugleich Säure- und Epoxygruppen im gleichen Molekül aufweist. Dadurch wird in der Applikation eine zweite thermische Vernetzungsreaktion zwischen diesen beiden Funktionalitäten ermöglicht Problematisch ist hier jedoch neben der Herstellung der Produkte (Gefahr der Gelierung bei der Reaktion mit dem Anhydrid) die Lagerstabilität, da bereits bei Raumtemperatur eine bestimmte Reaktivität der derartige Umsetzungsprodukte enthaltenden Formulierung vorhanden ist Generell sind all diese genannten Epoxyacrylate relativ niedermolekular.

Photochemisch oder thermisch gehärtete Epoxyacrylate, die sich von niedermolekularen Epoxyharzen und Epoxynovolaken ableiten, sind zwar für ihre guten thermischen und mechanischen Eigenschaften sowie ihre gute chemische Beständigkeit gegen agressive Chemikalien bekannt. Allerdings lassen aber die Klebrigkeit und die Kantendeckung der mit diesen Systemen erhaltenen Resistfilme auf Leiterzügen wegen der relativ niedrigen Molmasse zu wünschen übrig. Man ist deshalb in der Anwendung vielfach gezwungen, diese Nachteile durch den Zusatz von hochmolekularen Bindemittelpolymeren zu umgehen. Diese weisen normalerweise keine funktionellen Acrylatgruppen auf und reagieren bei der photochemischen oder thermischen Härtung nicht mit, d.h. sie werden als "passive" Bestandteile im Netzwerk nicht eingebaut und führen somit zu einer Verdünnung der Netzwerkdichte. Dies hat wieder eine ungünstige Beeinflussung speziell der chemischen Beständigkeit und der elektrischen Eigenschaften von prozessierten Resistschichten zur Folge. Ausserdem sinkt die Photoempfindlichkeit infolge der "Verdünnung" der Acrylatgruppen. Durch die Verwendung von hochmolekularen Bindemittelpolymeren weisen diese Zusammensetzungen bereits bei relativ niedrigem Festkörpergehalt eine hohe Viskosität auf und führen daher oft zu grossen Problemen bei der Beschichtung.

Aufgabe der vorliegenden Erfindung war es daher, Acrylate zu entwickeln, die die aufgezeigten Nachteile nicht aufweisen.

Erfindungsgemäss wird diese Aufgabe durch neue Epoxyacrylate und neue carboxylgruppenhaltige Epoxyacrylate gelöst, die höhermolekular sind und z.B. bei Verwendung in Resistformulierungen ohne oder nur mit geringen Mengen von zusätzlichen Binderpolymeren (Bindemittelpolymeren) auskommen. Sie werden durch Umsetzung von sogenannten "postglycidylisierten" Epoxyharzen (PGEH) mit z.B. (Meth)acrylsäure erhalten.

Batzer und Zahir (J. AppL Polym. Sci., 19, 609 (1975)) beschreiben die Postglycidylisierungsreaktion eines niedermolekularen, flüssigen Bisphenol A-diglycidylethers. Die US 4,623,701 beschreibt postglycidylisierte Epoxyharze und ihre Härtung mit diversen Epoxyhärtern, und die US 4 074 008 offenbart photovernetzbare Epoxyharze mit mehr als 2 Epoxygruppen im Molekül, wobei mindestens 2 davon aus einer Postglycidylisierungsreaktion stammen. Die in der Molekülkette angeordneten photovernetzbaren Gruppen stellen α,β-ungesättigte Carbonylsysteme dar (Chalcongruppen). (Meth)acrylgruppen enthaltende Derivate sind dort jedoch keine beschrieben.

Es ist ferner bekannt, dass die Photoempfindlichkeit des nach einem 2+2-Cycloadditionsmechanismus lichtvernetzbaren α,β-ungesättigten Carbonylsystems im Vergleich zur Photopolymerisation von Acrylaten wesentlich geringer ist.

In der japanischen Patentanmeldung Kokai Hei 04-294352 werden Epoxynovolakharze

Durch Umsetzung mit einer ungesättigten Monocarbonsäure und anschliessend mit einem ungesättigten Anhydrid einer Polycarbonsäure modifiziert und in photoempfindlichen wässrigen Zusammensetzungen eingesetzt. Ausserdem sind in der europäischen Patentanmeldung 0292219 photoempfindliche Systeme beschrieben, welche Bisphenol-A-Epoxyverbindungen enthalten, die mit Acrylsäure modifiziert sind. Schliesslich werden gemäss JP-A-5321746 carboxylgruppenhaltige Epoxyacrylate mit Polycarbonsäureanhydriden, wie Maleinsäureanhydrid oder Phthalsäureanhydrid, modifiziert und z. B. als Lötstoppresists vorgeschlagen.

Ein Gegenstand der Erfindung sind carboxylgruppenhaltige Epoxyacrylate der Formel IV 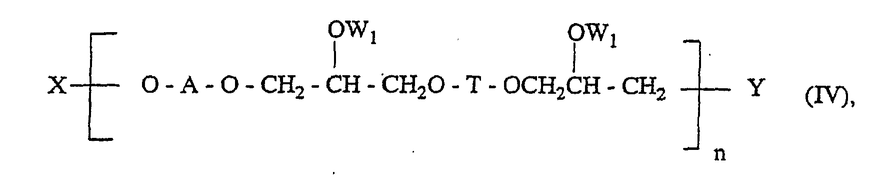 worin
- X: Wasserstoff oder eine Gruppe der Formeln 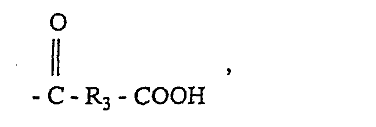 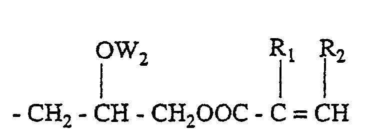 oder 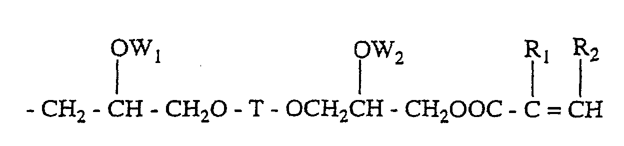 bedeutet,
- R₃: den Rest des Bernsteinsäureanhydrid nach Entfernung des Anhydridrestes,
- W₁: Wasserstoff oder eine Gruppe der Formeln 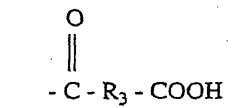 oder 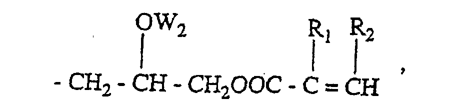
- W₂: -H oder die Gruppe 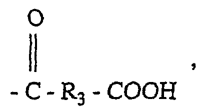 und
- Y: die Gruppe der Formeln -O-A-O-W₁ oder 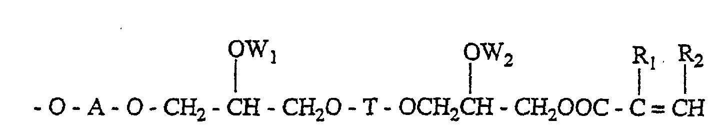 bedeuten, und worin
A und T den Rest einer bifunktionellen aromatischen Verbindung,
R₁ -H oder -CH₃,
R₂ -H, -CH₃ oder Phenyl, und
n eine ganze Zahl von 0 bis 300 bedeuten,
wobei in der Formel IV mindestens 10 Mol % der Reste W₁, die nicht in den Endgruppen X und Y sind, eine Gruppe der Formel 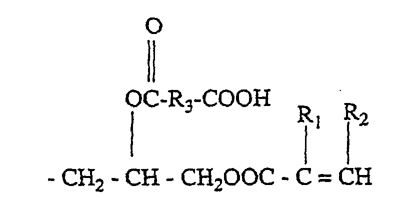 darstellen, wobei R₁, R₂ und R₃ die zuvor angebenen Bedeutungen haben.

Die erfindungsgemässen carboxylgruppenhaltigen Epoxyacrylate sind erhältlich aus Epoxyacrylaten der Formel III 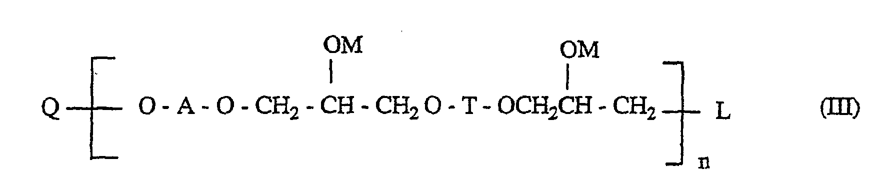 entwickelt, worin
- Q: Wasserstoff oder eine Gruppe der Formeln 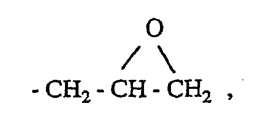 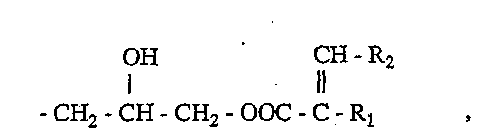 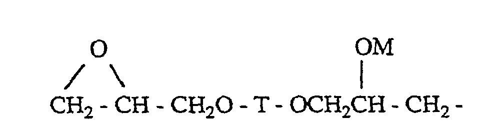 oder 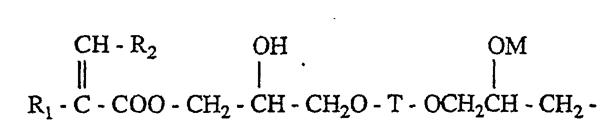 bedeutet,
- R₁: -H oder -CH₃, R₂ -H, -CH₃ oder Phenyl
- T: den Rest einer aromatischen bifunktionellen Verbindung, und
- M: unabhängig voneinander Wasserstoff oder eine Gruppe der Formeln 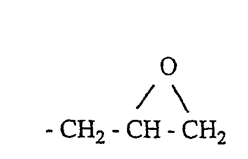 oder 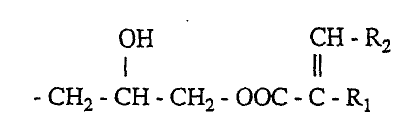 bedeuten, wobei
- R₁ und R₂: die oben angegebene Bedeutung haben,
- A: den Rest einer aromatischen bifunktionellen Verbindung,
- n: eine ganze Zahl von 0 bis 300, und
- L: eine Gruppe der Formeln 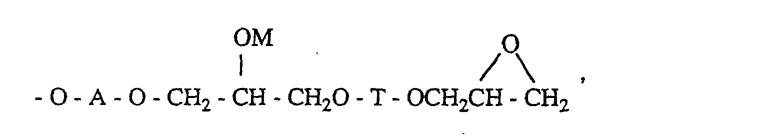 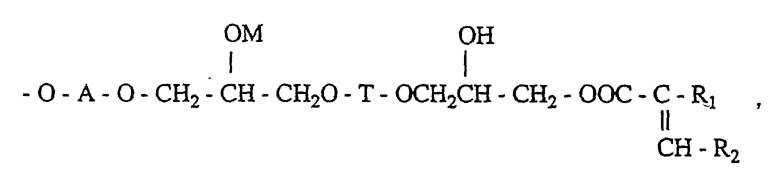 oder - O - A - OM bedeuten,
wobei in der Formel III nicht alle Reste M gleichzeitig Wasserstoff oder eine Gruppe der Formel 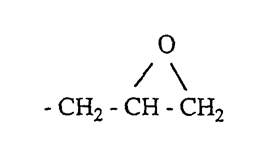 bedeuten können, sondern mindestens 10 Mol %, vorzugsweise 20-100 Mol % der Reste M, die nicht in den Endgruppen Q und L sind, eine Gruppe der obigen Formel 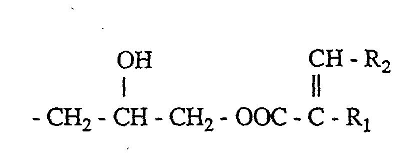 darstellen.

Die Epoxyacrylate der Formel III werden dadurch erhalten, dass ein postglycidylisiertes Epoxyharz der Formel II 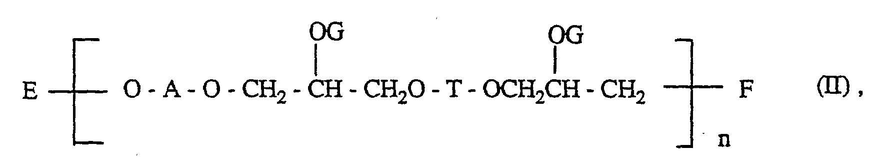 worin
- E: Wasserstoff oder eine Gruppe der Formeln 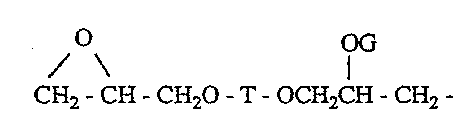 oder 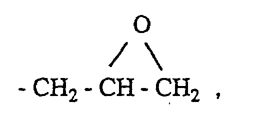
- F: die Gruppen der Formeln - O - A - OG oder 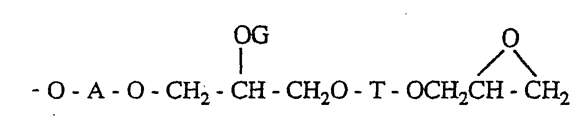 und
- G: -H oder der Rest 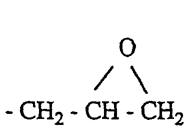 bedeuten, und
wobei analog zur Formel III mindestens 10 Mol % der Reste G in der Formel II, die nicht in den Endgruppen E und F sind, die Gruppe der Formel 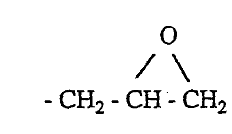 bedeuten, und
A, T, und n die angegebene Bedeutung haben,
mit einer ethylenisch ungesättigten Monocarbonsäure in Anwesenheit eines Katalysators und eines Polymerisationsinhibitors bei erhöhter Temperatur umgesetzt wird.

Für den Fall, dass n in der Formel III Null ist, bedeuten Q -H und L die Gruppe der Formel

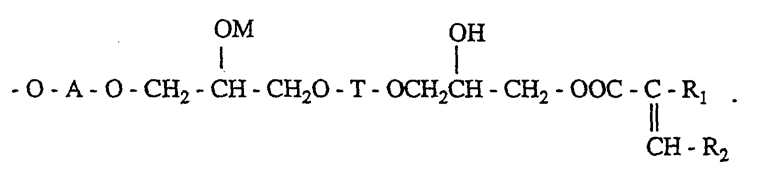

In bevorzugten Epoxyacrylaten der Formel III bedeutet n eine ganze Zahl von 0 bis 50, vor allem von 0 bis 30, und die Symbole A und T haben die in der japanischen Patentanmeldung Hei 1-195056 bevorzugten Bedeutungen von A und B.

Bevorzugte bifunktionelle aromatische Verbindungen A und T sind Brückenglieder der Formeln 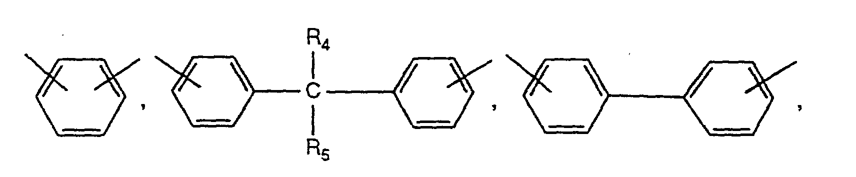 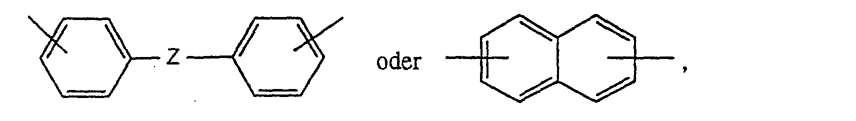 wobei R₄ und R₅ unabhängig voneinander -H oder C₁-C₄-Alkyl, Z -S-, -O- oder -SO₂ sind, und die aromatischen Reste des Brückengliedes A oder T unsubstituiert oder durch Halogen oder C₁-C₄-Alkyl substituiert sind. C₁-C₄-Alkyl ist besonders -CH₃, und Halogen bedeutet insbesondere Brom.

Besonders bevorzugte Brückenglieder A und T unabhängig voneinander entsprechen der Formel 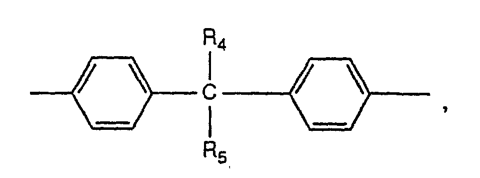 worin R₄ und R₅ die oben angegebene Bedeutung haben, und die Phenylreste des Brückengliedes unsubstituiert oder durch Brom substituiert sind, und vor allem den Formeln

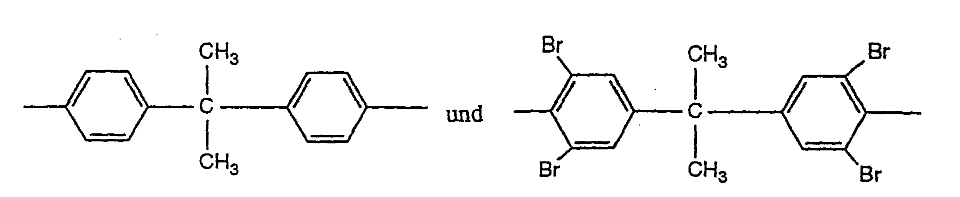

Als Epoxyacrylate werden hier und im folgenden Umsetzungsprodukte aus Epoxyverbindungen mit (Meth)acrylsäure bezeichnet.

Die postglycidylisierten Epoxyharze der Formel II sind zum Teil bekannt und werden aus den entsprechenden bekannten avancierten Epoxyharzen der Formel I 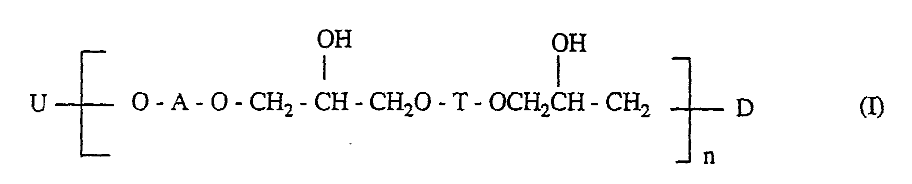 durch eine Glycidylisierungsreaktion hergestellt, worin in der Formel I
U Wasserstoff oder die Gruppe der Formel 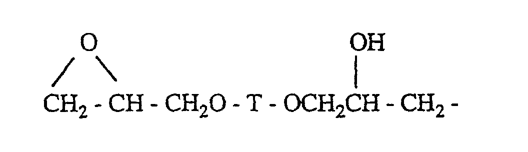 und
D die Gruppe der Formel 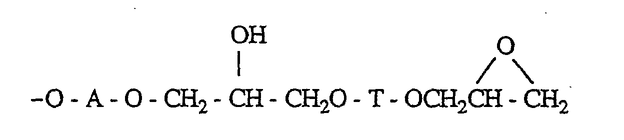 oder den Rest -O-A-OH bedeuten, wobei die Symbole A, T und n die unter der Formel III angegebene Bedeutung haben.

Die avancierten Epoxyharze der Formel I andererseits erhält man durch bekannte Polyaddition eines Bisphenols der Formel

HO-A-OH

mit einer Bisepoxyverbindung der Formel 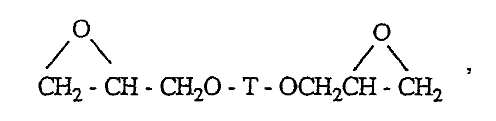 worin A und T den Rest einer bifunktionellen aromatischen Verbindung bedeuten.

Bei den Bisphenolen HO-A-OH oder HO-T-OH handelt es sich bevorzugt um bekannte Bisphenole, vor allem um Bisphenol A und Tetrabrom-Bisphenol A, sowie um solche, die in der japanischen Patentanmeldung Hei 1-195056 beschrieben sind, insbesondere Bisphenol A, Bisphenol F, Tetrabrombisphenol A und Tetrabrombisphenol F.

Normalerweise werden zur Herstellung der avancierten Epoxyharze die oben angegebenen Bisepoxyverbindungen im Ueberschuss eingesetzt, so dass die avancierten Epoxyharze der Formel I Epoxyendgruppen aufweisen. Es ist aber auch möglich, das Bisphenol HO-A-OH im Ueberschuss einzusetzen und damit Moleküle mit phenolischen Endgruppen herzustellen. Das Molekulargewicht wird durch das molare Verhältnis von Bisphenol HO-A-OH zur Bisepoxyverbindung bestimmt. Gegebenenfalls können auch kleine Mengen an höherfunktionellen Phenolen oder Epoxyverbindungen (z.B. Trisphenole oder Trisepoxyphenylverbindungen) der Polyaddition zugesetzt werden. Es können auch gewisse Mengen der Ausgangsprodukte (Bisphenol HO-A-OH und/oder Bisepoxyverbindung) im avancierten Epoxyharz vorhanden sein.

Die avancierten Epoxyharze der Formel I weisen sekundäre, aliphatische Hydroxylgruppen auf, die sich aus der Additionsreaktion der phenolischen Hydroxylgruppe mit dem Oxiranring 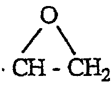 ergeben.

Die Glycidylisierungsreaktion zu den postglycidylisierten Epoxyharzen der Formel II erfolgt nach bekannten Methoden, indem das avancierte Epoxyharz (I) mit z.B. Epichlorhydrin im Ueberschuss in Gegenwart einer Base (z.B. NaOH) und eines Katalysators bei erhöhter Temperatur zur Reaktion gebracht wird.

Die Menge der eingesetzten Base in der Glycidylisierungsreaktion richtet sich nach dem gewünschten Glycidylisierungsgrad; vorzugsweise werden 0,1 bis 1,2 Aequivalente Base pro Aequivalent sekundäre HO-Gruppe im avanzierten Epoxyharz verwendet. Wasser wird durch azeotrope Destillation mit überschüssigem Epichlorhydrin als Schleppmittel entfernt.

Als Katalysatoren kommen vor allem quaternäre Ammoniumsalze oder Phosphoniumsalze, wie Tetramethylammoniumchlorid, Benzyltrimethylammoniumchlorid, Benzyltriethylammoniumchlorid, Tetraethyl- und Tetrabutylammoniumbromid, in Frage.

Die Reaktionstemperatur liegt zweckmässig etwa zwischen 40 bis 80°C, vorzugsweise zwischen 50 und 65°C.

Durch die Glycidylisierungsreaktion werden die aliphatischen OH-Gruppen teilweise oder vollständig glycidylisiert.

Die weitere Umsetzung der postglycidylisierten Epoxidharze der Formel II zu Epoxyacrylaten der Formel III erfolgt ebenfalls auf bekannte Art und Weise, durch Reaktion mit einer ethylenisch ungesättigten Monocarbonsäure der Formel

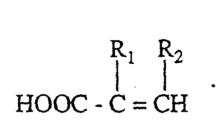

Es kommen z.B. Crotonsäure, Zimtsäure und vor allem Acrylsäure oder Methacrylsäure oder deren Mischung in Betracht. R₁ und R₂ haben die oben angegebene Bedeutung.

Bei der Reaktion wird bevorzugt ein Katalysator eingesetzt. Als Katalysatoren kommen vor allem Metallsalze, wie z.B. Chromverbindungen, Amine, wie Triethylamin oder Benzyldimethylamin, ferner Ammoniumsalze, wie z.B. Benzyltrimethylammoniumchlorid, oder dann auch Triphenylphosphin und Triphenylwismuth, in Frage.

Gegebenenfalls wird der Reaktion ein Lösungsmittel zugefügt, da die postglycidylisierten Epoxyharze der Formel II als Feststoffe vorliegen. Das Lösungsmittel muss jedoch gegenüber dem Edukt inert sein. Als Lösungsmittel kommen z.B. in Frage: Ketone, wie Aceton, Methylethylketon, Cyclohexanon; Ester wie Essigsäureethyl- und -butylester, Ethoxyethylacetat oder Methoxypropylacetat; Ether, wie Dimethoxyethan und Dioxan; aromatische Kohlenwasserstoffe, wie Toluol, Benzol und Xylole, sowie Gemische von zwei oder mehreren dieser genannten Lösungsmittel.

Die Temperatur variiert zweckmässig zwischen 80 und 140°C, wobei die Reaktion mit Acrylsäure bevorzugt bei 80 bis 120°C und mit Methacrylsäure bevorzugt bei 80 bis 140°C durchgeführt wird.

Gegebenenfalls kann dem Reaktionsmedium auch ein Polymerisationsinhibitor zugesetzt werden; als solche kommen z.B. in Frage: Hydrochinon, Hydrochinonmonomethylether und 2,6-Di-tert.-butyl-p-kresol.

Es ist zweckmässig, in das Reaktionsmedium Luft oder ein Gemisch aus Stickstoff/Sauerstoff einzuleiten, da einige der oben genannten Polymerisationsinhibitoren nur in Anwesenheit von Sauerstoff wirksam sind. In Abhängigkeit der eingesetzten Menge der ethylenisch ungesättigten Monocarbonsäure werden Epoxyacrylate der Formel III erhalten, die vollständig oder nur teilweise acryliert sind. Dabei kann die Monocarbonsäure in aequimolaren Mengen bezüglich der Epoxygruppen oder im Unterschuss eingesetzt werden. Die vollständig umgesetzten Epoxyacrylate enthalten praktisch keine Epoxygruppen mehr.

Die erfindungsgemässen Epoxyacrylate der Formel III müssen weder aus dem Reaktionsmedium isoliert noch gereinigt werden. Die erhaltene Reaktionslösung kann als solche verwendet werden.

Sowohl die teilweise als auch die vollständig umgesetzten Produkte der Formel III enthalten aliphatische Hydroxylgruppen, die aus der Reaktion der Epoxygruppen mit der ethylenisch ungesättigten Monocarbonsäure herrühren. Zusätzlich können noch aliphatische Hydroxylgruppen aus dem Edukt vorhanden sein.

Die vollständig acrylierten Epoxyacrylate der Formel III können dann zu carboxylgruppenhaltigen Epoxyacrylaten der Formel IV 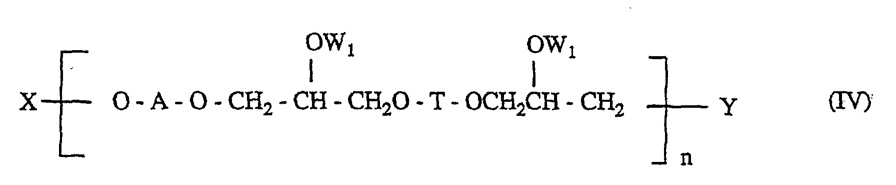 weiter umgesetzt werden, worin
- X: Wasserstoff oder eine Gruppe der Formeln 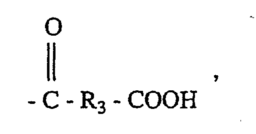 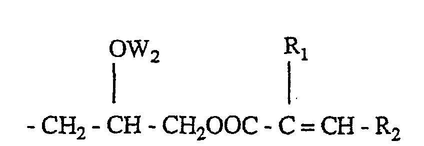 oder 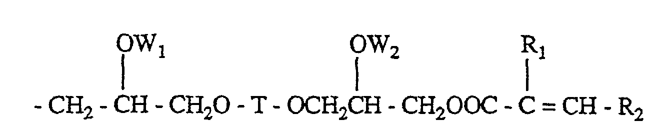 bedeutet,
- R₃: den Rest des Bernsteinsäureanhydrids nach Entfernen des Anhydridrestes,
- W₁: Wasserstoff oder eine Gruppe der Formeln 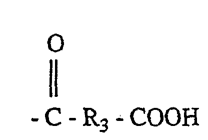 oder 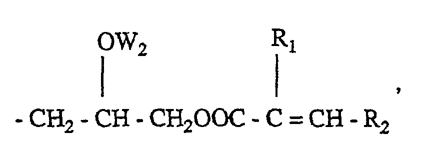
- W₂: -H oder die Gruppe 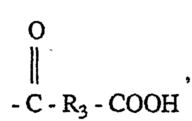 und
- Y: die Gruppe der Formel -O-A-O-W₁ oder 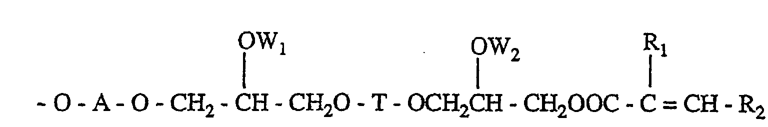 bedeuten,
worin die Symbole A, T, R₁, R₂, R₃ und n die oben angegebene Bedeutung haben, wobei in der Formel IV mindestens 10 Mol % der Reste W₁, die nicht in den Endgruppen X und Y sind, eine Gruppe der Formel 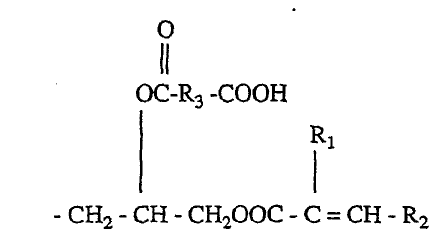 darstellen.
R₁, R₂ und R₃ haben die oben angegebene Bedeutung.
Da die vollständig umgesetzten Epoxyacrylate der Formel III praktisch keine Epoxygruppen mehr enthalten, lassen sie sich mit cyclischen Anhydriden von Polycarbonsäuren zur Reaktion bringen. Dabei reagieren die aliphatischen Hydroxylgruppen mit dem cyclischen Anhydrid unter Ringöffnung und Halbesterbildung. Pro umgesetzte Hydroxylgruppe entsteht dabei eine an das Harz gebundene Carbonsäure. Die Reaktion geschieht derart, dass das Epoxyacrylat der Formel III mit Bernsteinsäureanhydrid, gegebenenfalls in Anwesenheit eines Katalysators und eines Polymerisationsinhibitors, bei erhöhter Temperatur umgesetzt wird. Die HO-Gruppen der Verbindungen der Formel II werden dabei unter Ringöffnung des Anhydrides ganz oder teilweise acyliert. Es ist daher vorteilhaft, dass die Epoxyacrylate der Formel III keine Epoxygruppen mehr aufweisen, ansonsten eine Gelbildung eintritt. Das Anhydrid wird in aequimolaren Mengen bezüglich der Hydroxylgruppen oder im Unterschuss eingesetzt. Die Reaktion ist als solche bekannt.

Als Katalysator kommen z.B. Amine, wie z.B. Triethylamin, Benzyldimethylamin, Pyridin oder Dimethylaminopyridin, oder Triphenylphosphin oder Metallsalze, wie Chrom- oder Zirkoniumverbindungen, in Frage.

Gegebenenfalls kann dem Reaktionsmedium ein Lösungsmittel zugesetzt werden, da die Epoxyacrylate der Formel III als Feststoffe vorliegen. Dieses muss aber gegenüber dem cyclischen Anhydrid inert sein; daher kommen z.B. Hydroxylgruppen aufweisende Lösungsmittel nicht in Frage. Als Lösungsmittel kommen z.B. die für die Reaktion mit den ethylenisch ungesättigten Monocarbonsäuren oben genannten Lösungsmittel in Betracht.

Die Reaktionstemperatur bewegt sich zweckmässig zwischen 60 und 140°C, und als Polymerisationsinhibitoren kommen z.B. Hydrochinon, Hydrochinonmonomethylether und 2,6-Di-tert.-butyl-p-kresol in Frage.

Es ist vorteilhaft in das Reaktionsmedium trockene Luft oder ein Stickstoff/Sauerstoff-Gemisch einzuleiten. In einer bevorzugten Ausführungsform der Erfindung werden die Epoxyacrylate der Formel III ohne Isolierung in der gleichen Reaktionsapparatur zu den mit Carboxylgruppen modifizierten Derivaten der Formel IV weiter umgesetzt.

Eine Isolierung und Reinigung der neuen carboxylgruppenhaltigen Epoxyacrylate der Formel IV ist in der Regel nicht nötig; die Reaktionslösung kann als solche weiterverwendet werden.

Infolge der im Molekül vorhandenen ungesättigten Gruppen sind die Epoxyacrylate der Formel III und die carboxylgruppenhaltigen Epoxyacrylate der Formel IV thermisch und photochemisch vernetzbar. Sie können deshalb als Acrylatkomponenten z.B. in Photoresistformulierungen für die Herstellung von Lötstoppresists oder Primärresists nach bekannten Verfahren verwendet und appliziert werden, wie z.B. in der am 2. Juli 1993 hinterlegten Schweiz. Patentanmeldung Nr. 2005/93-4 "Photopolymerisierbare Zusammensetzungen", und ergeben Resistschichten mit verbesserten thermischen, mechanischen, elektrischen und chemischen Eigenschaften. Die daraus hergestellten Resistformulierungen werden vor allem auf dem Gebiet der Leiterplatten z.B. als Lötstoppresist oder Primärresist, und der Druckplatten angewandt. Des weiteren kommen sie zur Herstellung von Offsetdruckplatten, Flexodruckplatten, Buchdruckplatten und Siebdruckformulierungen in Frage. Als Entwickler kommen sowohl wässrige als auch wässrig/organische oder organische Systeme in Frage. Wegen dem Vorliegen von Carboxylgruppen in den Verbindungen der Formel IV sind diese vor allem für die Herstellung von wässrig-alkalisch entwickelbaren Photoresists geeignet.

Gegenüber niedermolekularen Epoxyacrylaten in Formulierungen, die Binderpolymere enthalten, ist es überraschend, dass Formulierungen mit höhermolekularen Epoxyacrylaten ohne Zusatz derartiger Binderpolymere keinen Verlust an sondern eine Verbesserung der Photoempfindlichkeit aufweisen, und auch dass keine Erhöhung der Klebrigkeit erfolgt. Des weiteren ist eine bessere Kantendeckung der Leiterzüge bei ihrer Verwendung als Lötstoppresist gegeben. Da in derartigen Formulierungen keine zusätzlichen Binderpolymere eingesetzt werden, können weitere Vorteile bezüglich der thermischen, mechanischen und elektrischen Eigenschaften und insbesondere bezüglich der chemischen Beständigkeit der daraus gewonnenen Resistzusammensetzungen erhalten werden. Ferner weisen die erfindungsgemässen Epoxyacrylate der Formel III und die carboxylgruppenhaltigen Epoxyacrylate der Formel IV eine erhöhte Glasumwandlungstemperatur auf.

Die folgenden Beispiele veranschaulichen die Erfindung, ohne sie darauf zu limitieren.

### Herstellungsbeispiele

### a) Postglycidylisierte Epoxyharze (PGEH)

Beispiel 1: Es wird eine für Tetramethylammoniumchlorid (TMAC)-Glycidylisierungen geeignete Apparatur verwendet, die es gestattet, überschüssiges Epichlorhydrin als Schleppmittel für die Wasserabscheidung unter vermindertem Druck einzusetzen. Es handelt sich dabei um ein 5000 ml Reaktionsgefäss mit Rührer, Thermometer, einem gut wirksamen Intensivkühler und 2 Tropftrichtern mit Druckausgleich. Angeschlossen sind ein Wasserabscheider (für obenliegende wässrige Phase und Entleerung unter Vakuum) und eine Wasserstrahlpumpe mit Manometer. Die Heizung geschieht mittels Oelbad.

1000 g Epoxidharz Araldit GT 7004 der Firma CIBA (Epoxygruppengehalt: 1,36 Mol/kg Harz; HO-Gruppen-Gehalt: 2,71 Mol) und 1700 ml (21,68 Mol) Epichlorhydrin werden im Reaktor durch Erwärmen gelöst. Die erhaltene homogene Lösung wird auf eine Innentemperatur von ca. 80°C erwärmt, und unter guter Rührung wird das Wasserstrahlvakuum so vorsichtig angelegt, dass das Epichlorhydrin kräftig am Rückfluss siedet. Die Innentemperatur sinkt ab und wird auf einen konstanten Wert von ca. 55°C eingestellt und während der gesamten Reaktion gehalten. Dies bedingt anfänglich einen Druck von 110-120 mbar, den man gegen Schluss hin eventuell etwas senken muss. Die Oelbadtemperatur beträgt konstant 105-110°C. Wenn das Epichlorhydrin bei 55°C konstant rückflussiert, tropft man aus dem ersten Tropftrichter 29,80 g (0,136 Mol) einer 50%-igen wässrigen Tetramethylammonium-chloridlösung rasch zu. Dann werden innnerhalb von 2 Stunden aus dem zweiten Tropftrichter 195,20 g (2,44 Mol) einer 50%-igen wässrigen Natriumhydroxydlösung zugetropft, und gleichzeitig werden das entstehende Wasser sowie das aus den 50%-Lösungen durch azeotrope Destillation stammende Epichlorhydrin ausgekreist. Die Innentemperatur hält man bei 55-58°C und sorgt durch genügend Rückfluss für eine zügige Entfernung des Wassers aus dem Reaktionsgemisch. Man lässt nach dem Zutropfen noch 2 Stunden bei 55°C unter Wasserauskreisung nachreagieren.
Das Vakuum wird abgestellt, und die Suspension wird durch Zugabe von zerkleinertem Trockeneis, gefolgt von 10-20 ml Eisessig, neutralisiert. Nach dem Zufügen von 2000 ml Methoxypropylacetat werden das Lösungsmittel und das Epichlorhydrin unter vermindertem Druck abdestilliert. Zum Rückstand gibt man nochmals 2000 ml Methoxypropylacetat und filtriert dann die erhaltene Suspension über ein Filtrationshilfsmittel (Hyflo), wobei man von Zeit zu Zeit die Oberfläche des Filterbetts aufkratzt. Das klare, gelbliche Filtrat engt man am Rotationsverdampfer bei einer Badetemperatur von bis zu 120°C ein. Das gewonnene gelbe, klare Harz wird durch Zugabe von Methoxypropylacetat zu einer Lösung mit 50% Festkörpergehalt verdünnt. Man erhält 2316 g einer 50%-igen, praktisch farblosen Lösung des postglycidylisierten Epoxyharzes der Formel II, worin
- A: 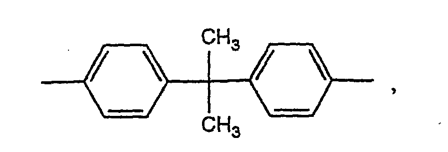
- T: 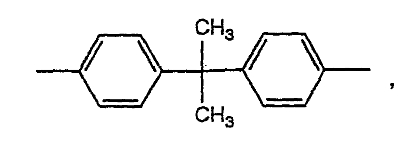
- E: 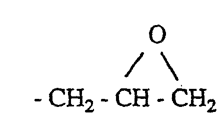 und
- F: 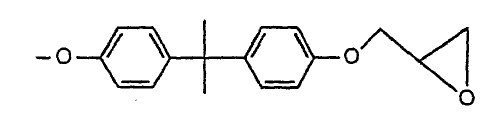 bedeuten, und
- G: etwa 90 Mol% der Gruppe 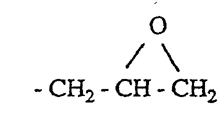 und 10 Mol% -H bedeutet, und
- n: einen Mittelwert von 2 hat.

Diese Lösung kann direkt für die Umsetzung mit Acrylsäure eingesetzt werden. Analytische Daten dieser Lösung:

| | | |
|---|---|---|
| 1. | Gehaltsbestimmung (Trockengewicht) | 49,5%; |
| 2. | Epoxywert (titrimetrisch bestimmt) | 1,46 Mol/kg/(→2,81 Mol/kg Festharz); |
| 3. | Chlorgehalt (Festharz) | 0,40% Chlor total; |
| | | 0,23% Chlor hydrolysierbar; |
| 4. | GPC (Gelpermeationschromatographie Polystyrol-Eichung) | M_{w} = 10678; Mₙ = 2138. |

Falls das Festharz gewünscht wird, kann man bei der Aufarbeitung anstelle von Methoxypropylacetat als Lösungsmittel Methylisobutylketon zum Verdünnen einsetzen und den Rückstand nach dem Abrotieren (Einengen im Rotationsverdampfer) heiss in flache Stahlwannen giessen und im Hochvakuum bei 150°C mehrere Stunden trocknen.

Beispiel 2: Gemäss dem Verfahren des Beispiels 1 werden 555 g Araldit GT 7004 der Firma CIBA (Epoxygruppengehalt: 1,36 Mol/kg Harz; HO-Gruppengehalt: 1,50 Mol), 705 ml Epichlorhydrin (9,00 Mol), 16,40 g einer 50%-igen wässrigen Tetramethylammoniumchloridlösung und 66,00 g einer 50%-igen wässrigen Natriumhydroxydlösung (0,83 Mol) umgesetzt. Nach dem Zutropfen der Natronlauge lässt man noch 45 Min. unter Wasserabscheidung nachreagieren und destilliert dann bei einem Vakuum von ca. 85 mbar den Grossteil des Epichlorhydrins ab. Man fügt 600 ml Methylisobutylketon zu, neutralisiert mit Trockeneis und Essigsäure und verdünnt mit weiteren 500 ml Methylisobutylketon. Die Suspension wird über ein Filterhilfsmittel (Hyflo) filtriert, das Lösungsmittel wird abrotiert, der Rückstand wird heiss in Stahlwannen gegossen und am Hochvakuum bei 150°C mehrere Stunden getrocknet. Analytische Daten des erhaltenen leicht gelblichen Festharzes (473 g):

| | | |
|---|---|---|
| 1. | Epoxywert (titrimetrisch) | 2,39 Mol/kg Harz; |
| | | |
| 2. | Chlorgehalt | 0,33% Chlor total; 0,17% Chlor hydrolysierbar; |
| | | |
| 3. | OH-Gruppengehalt (titrimetrisch) | 1,22 Mol/kg; |
| | | |
| 4. | GPC (Polystyrol-Eichung) | M_{w} = 10721; Mₙ = 2330. |

Das Festharz entspricht der Formel II, worin A und T sowie E, F und n die im Beispiel 1 angegebene Bedeutung haben und
- G: etwa 55 Mol% der Gruppe 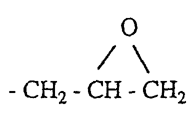 und 45 Mol% -H bedeutet.

Beispiel 3: Nach dem Verfahren gemäss Beispiel 1 werden *525* g Araldit B 41 der Firma CIBA (Epoxygruppengehalt: 2,66 Mol/kg Harz; HO-Gruppengehalt: 1,01 Mol), 712 ml Epichlorhydrin (9,09 Mol), 18,48 g einer 50%-igen wässrigen Tetramethylammoniumchloridlösung und 89,00 g einer 50%-igen wässrigen Natriumhydroxydlösung (1,11 Mol) umgesetzt und als Festharz isoliert
Analytische Daten des praktisch farblosen Festharzes (515 g):

| | | |
|---|---|---|
| 1. | Epoxywert (titrimetrisch) | 3,48 Mol/kg; |
| | | |
| 2. | Chlorgehalt | 0,21% Chlor total; |
| | | |
| | | <0,05% Chlor hydrolysierbar; |
| 3. | GPC (Polystyrol-Eichung) | M_{w} = 2374; Mₙ = 935. |

Das Festharz entspricht der Formel II, worin T, E und F die im Beispiel 1 angegebene Bedeutung haben, n einen Mittelwert von etwa 0,7 aufweist und
- G: 100 Mol% 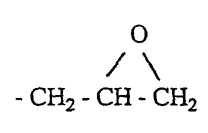 bedeutet.

Beispiel 4: Nach dem Verfahren gemäss Beispiel 1 werden 435 g Araldit GT 6071 der Firma CIBA (Epoxygruppengehalt: 2,18 Mol/kg Harz; HO-Gruppengehalt: 1,00 Mol), 470 ml Epichlorhydrin (6,00 Mol), 11,00 g einer 50%-igen wässrigen Tetramethylammoniumchloridlösung und 88,00 g einer 50%-igen wässrigen Natriumhydroxydlösung (1,10 Mol) umgesetzt und als Festharz isoliert.
Analytische Daten des leicht gelblichen Festharzes (390 g):

| | | |
|---|---|---|
| 1. | Epoxywert (titrimetrisch) | 3,55 Mol/kg; |
| | | |
| 2. | Chlorgehalt | 0,82% Chlor total; |
| | | |
| | | 0,60% Chlor hydrolysierbar; |
| 3. | GPC (Polystyrol-Eichung) | M_{w} = 6498; Mₙ = 1376. |

Das Festharz entspricht der Formel II, worin A, T, E und F die im Beispiel 1 angegebene Bedeutung haben und n einen Mittelwert von 1,0 aufweist und G 100 Mol% 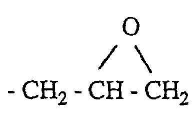 bedeutet

Beispiel 5: Nach dem Verfahren gemäss Beispiel 1 werden 1000 g Araldit GT 6097 der Firma CIBA (Epoxygruppengehalt: 0,60 Mol/kg Harz; HO-Gruppengehalt: 3,175 Mol), 1996 ml Epichlorhydrin (25,40 Mol), 35,20 g einer 50%-igen wässrigen Tetramethylammoniumchloridlösung und 228,60 g einer 50%-igen wässrigen Natriumhydroxydlösung (2,86 Mol) umgesetzt und als ca. 50%-Lösung in Methoxypropylacetat isoliert
Analytische Daten dieser Lösung (2283 g):

| | | |
|---|---|---|
| 1. | Festkörpergehalt | 48,30%; |
| | | |
| 2. | Epoxywert (titrimetrisch) | 1,17 Mol/kg; |
| | | |
| 3. | Chlorgehalt | 0,63% Chlor total; |
| | | |
| | | 0,30% Chlor hydrolysierbar; |
| 4. | GPC (Polystyrol-Eichung) | M_{w} = 16759; Mₙ = 3382. |

Der Festkörper in der Lösung entspricht der Formel II, worin A, T, E und F die im Beispiel 1 angegebene Bedeutung haben, n einen Mittelwert von 5,3 aufweist und
- G: 90 Mol% 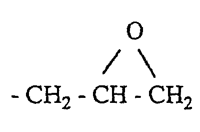 und 10 Mol% -H bedeutet.

Beispiel 6: Nach dem Verfahren gemäss Beispiel 1 werden 500 g Araldit GT 7097 der Firma CIBA (Epoxygruppengehalt: 0,58 Mol/kg Harz; HO-Gruppengehalt: 1,60 Mol), 753 ml Epichlorhydrin (9,60 Mol), 17,54 g einer 50%-igen wässrigen Tetramethylammoniumchloridlösung und 115,20 g einer 50%-igen wässrigen Natriumhydroxydlösung (1,44 Mol) umgesetzt und als ca. 45%-Lösung in Methoxypropylacetat isoliert Analytische Daten dieser Lösung (1200 g):

| | | |
|---|---|---|
| 1. | Festkörpergehalt | 44,50%; |
| | | |
| 2. | Epoxywert (titrimetrisch) | 1,19 Mol/kg ; |
| | | |
| 3. | Chlorgehalt | 0,70% Chlor total; |
| | | |
| | | 0,45% Chlor hydrolysierbar; |
| 4. | GPC (Polystyrol-Eichung) | M_{w} = 23091; Mₙ = 4270. |

Der Festkörper in der Lösung entspricht der Formel II, worin A, T, E und F die im Beispiel 1 angegebene Bedeutung haben, n einen Mittelwert von 5,5 aufweist und
- G: 90 Mol% 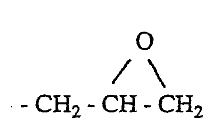 und 10 Mol% -H bedeutet.

Beispiel 7: Nach dem Verfahren gemäss Beispiel 1 werden 400 g Araldit GT 6099 der Firma CIBA (Epoxygruppengehalt: 0,41 Mol/kg Harz; HO-Gruppengehalt: 1,32 Mol), 1038 ml Epichlorhydrin (11,88 Mol), 14,08 g einer 50%-igen wässrigen Tetramethylammoniumchloridlösung und 95,04 g einer 50%-igen wässrigen Natriumhydroxydlösung (1,19 Mol) umgesetzt und als ca. 45%-Lösung in Methoxypropylacetat isoliert.
Analytische Daten dieser Lösung (915 g):

| | | |
|---|---|---|
| 1. | Festkörpergehalt | 45,60%; |
| | | |
| 2. | Epoxywert (titrimetrisch) | 1,16 Mol/kg ; |
| | | |
| 3. | Chlorgehalt | 0,42% Chlor total; |
| | | |
| | | 0,24% Chlor hydrolysierbar; |
| 4. | GPC (Polystyrol-Eichung) | M_{w} = 57017; Mₙ = 5604. |

Der Festkörper in der Lösung entspricht der Formel II, worin A, T, E und F die in Beispiel 1 angegebene Bedeutung haben,
- n: etwa 8,0 ist und
- G: 90 Mol% 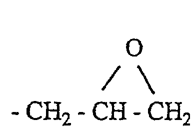 und 10 Mol% -H bedeutet.

Beispiel 8: Nach dem Verfahren gemäss Beispiel 1 werden 1171 g Araldit Festharz der Firma CIBA (Bromhaltiges Epoxyharz; Epoxygruppengehalt: 1,85 Mol/kg Harz; HO-Gruppengehalt: 2,26 Mol), 1416 ml Epichlorhydrin (18,10 Mol), 24,80 g einer 50%-igen wässrigen Tetramethylammoniumchloridlösung und 162,60 g einer 50%-igen wässrigen Natriumhydroxydlösung (2,03 Mol) umgesetzt und als Festharz isoliert.
Analytische Daten dieses Festharzes (1050 g):

| | | |
|---|---|---|
| 1. | Epoxywert (titrimetrisch) | 2,40 Mol/kg; |
| | | |
| 2. | Chlorgehalt | 0,45% Chlor total; |
| | | |
| | | 0,22% Chlor hydrolysierbar; |
| 3. | GPC (Polystyrol-Eichung) | M_{w} = 3189; Mₙ = 1235. |

Das Festharz entspricht der Formel II, worin
- A: 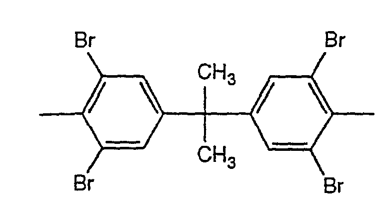 und
- T: 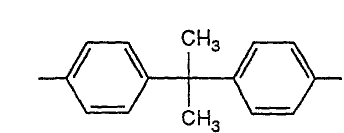 bedeuten, und
- E und F: die im Beispiel 1 angegebene Bedeutung haben, n etwa 1,0 ist und
- G: 90 Mol% 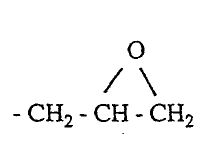 und 10 Mol% -H bedeutet

### b) Teilweise oder vollständig acrylierte Epoxyacrylate von PGEH

Beispiel 9: Als Apparatur verwendet man ein 5000 ml Reaktionsgefäss, ausgestattet mit Rührer, Tropftrichter, Thermometer, Rückflusskühler und einem Einleitungsrohr für Luft. Zur Inhibierung der Polymerisation der Acrylate wird während der Reaktion ein schwacher Luftstrom unter Niveau eingeleitet. Die Heizung geschieht mittels thermostatisierbarem Oelbad. Im Reaktor werden 2316,5 g einer 45%-igen Lösung (in Methoxypropylacetat) des Reaktionsproduktes gemäss Beispiel 1 (2,965 Mol Epoxygruppen), 3,80 g Hydrochinonmonomethylether und 5,20 ml Triethylamin vorgelegt und unter Rühren auf 95°C Innentemperatur aufgeheizt. Unter Einleitung eines schwachen Luftstromes dosiert man 213,70 g Acrylsäure (2,965 Mol) innerhalb einer Stunde bei einer Innentemperatur von 95°C zu und lässt 25 Stunden bei dieser Temperatur weiterreagieren. Die Reaktion wird durch Messen des Epoxywerts verfolgt; am Schluss beträgt er weniger als 0,08 Mol/kg. Das Reaktionsprodukt wird abgekühlt und ohne zusätzliche Reinigung weiterverwendet.

| | | |
|---|---|---|
| 1. | Festkörpergehalt | 54%; |
| | | |
| 2. | Säuregehalt (titrimetrisch) | 0,03 Mol/kg ; |
| | | |
| 3. | Viskosität (Brookfield) 25°C | 1970 mPa.s; |
| | | |
| 4. | GPC (Polystyrol-Eichung) | M_{w} = 8889; Mₙ = 2256. |

Der Festkörper entspricht der Formel III, worin A und T die im Beispiel 1 angegebene Bedeutung haben,
- Q: 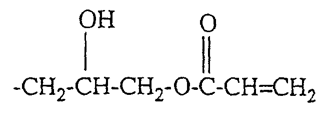 und
- L: 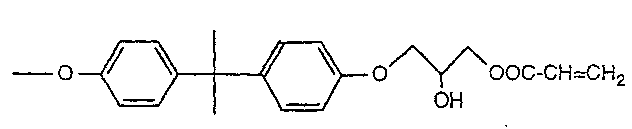 bedeuten und
- n: einen Wert von etwa 2,0 hat und
- M: 90 Mol% 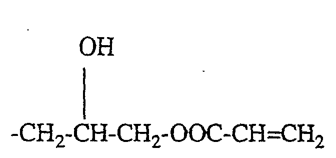 und 10 Mol% -H bedeutet

Beispiel 10: Als Apparatur verwendet man ein 2000 ml Reaktionsgefäss, ausgestattet mit Rührer, Thermometer, Rückflusskühler und einem Einleitungsrohr für Luft. Zur Inhibierung der Polymerisation der Acrylate wird während der Reaktion ein schwacher Luftstrom unter Niveau eingeleitet. Die Heizung geschieht mittels thermostatisierbarem Oelbad. Der Reaktor wird mit folgenden Komponenten beschickt und bei Raumtemperatur bis zum Erreichen der homogenen Lösung gerührt: 1018,60 g einer 50%-igen Lösung (in Methoxypropylacetat) des Reaktionsproduktes gemäss Beispiel 1 (1,422 Mol Epoxygruppen titriert); 102,46 g Acrylsäure (1,422 Mol); 1,53 g Hydrochinonmonomethylether; 20,38 ml einer 10%-igen Lösung (in Methoxypropylacetat) von Nuosynchromium 5 (Produkt der Firma HARCROS - Durham Chemicals, Durham DH3 1QX, GB).
Das Reaktionsgemisch wird auf eine Innentemperatur von 110°C aufgeheizt und bei dieser Temperatur unter Rühren und Einleiten eines schwachen Luftstroms während 7 Stunden gehalten. Das Fortschreiten der Reaktion lässt sich mittels Säure- oder Epoxytitration verfolgen. Nach 7 Stunden ergab die Säuretitration einen Wert von 0,05 Mol/kg.
Das Reaktionsprodukt wird nach dem Abkühlen abgefüllt und ohne weitere Reinigung weiterverwendet. Analytische Daten des Reaktionsproduktes (1080 g):

| | | |
|---|---|---|
| 1. | Festkörpergehalt | 53,3%; |
| | | |
| 2. | Epoxywert (titrimetrisch) | 0,02 Mol/kg; |
| | | |
| 3. | GPC (Polystyrol-Eichung) | M_{w} = 12168; Mₙ = 2602. |

Der Festkörper entspricht der chemischen Struktur gemäss Beispiel 9.

Beispiel 11: Apparatur (500 ml Reaktor) gemäss Beispiel 10.
Nach dem Verfahren von Beispiel 10 werden 200 g einer 50%-igen Lösung (in Methoxypropylacetat) des Reaktionsproduktes gemäss Beispiel 1 (0,292 Mol Epoxygruppen), 10,52 g Acrylsäure (0,146 Mol), 0,33 g Hydrochinonmonomethylether und 20,00 ml einer 1%-igen Lösung (in Methoxypropylacetat) von Nuosynchromium 5 umgesetzt. Die Reaktion ist nach 3 Stunden bei 100°C Innentemperatur beendet, die Säuretitration ergibt dann <0,02 Mol Säure.
Analytische Daten des Reaktionsproduktes (210 g):

| | | |
|---|---|---|
| 1. | Festkörpergehalt | 46%; |
| | | |
| 2. | Epoxywert (titrimetrisch) | 0,71 Mol/kg ; |
| | | |
| 3. | GPC (Polystyrol-Eichung) | M_{w} = 11036; Mₙ = 2352. |

Der Festkörper entspricht der Formel III, worin A und T die im Beispiel 1 angegebene Bedeutung haben,
- Q: 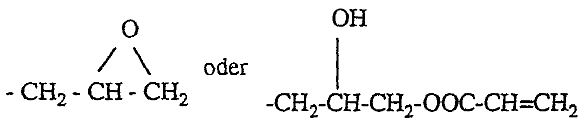 bedeutet,
- L: 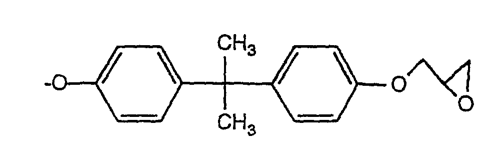 oder 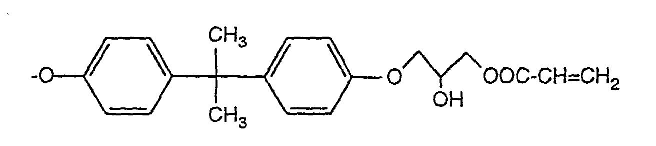
bedeuten und n einen Wert von 2 hat, und
- M: 90 Mol% 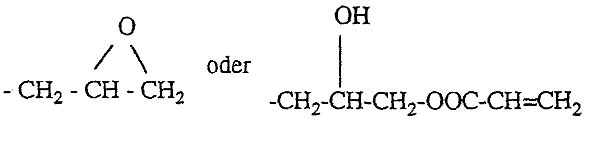 und 10 Mol%
-H ist.

Beispiel 12: Apparatur gemäss Beispiel 11.
Nach dem Verfahren von Beispiel 10 werden 200 g einer 50%-igen Lösung (in Methoxypropylacetat) des Reaktionsproduktes gemäss Beispiel 5 (0,236 Mol Epoxygruppen), 17,00 g Acrylsäure (0,236 Mol), 0,33 g Hydrochinonmonomethylether und 0,54 g Triphenylphosphin umgesetzt. Die Reaktion ist nach 14 Stunden bei 100°C Innentemperatur beendet, die Säuretitration ergibt dann 0,09 Mol Säure/kg.
Analytische Daten des Reaktionsproduktes (210 g):

| | | |
|---|---|---|
| 1. | Festkörpergehalt | 54%; |
| | | |
| 2. | Epoxywert (titrimetrisch) | 0,10 Mol/kg; |
| | | |
| 3. | GPC (Polystyrol-Eichung) | M_{w} = 19493; Mₙ = 3075. |

Der Festkörper entspricht der Formel III, worin A und T die im Beispiel 1 und Q, L, M die im Beispiel 9 angegebene Bedeutung haben und n einen Wert von etwa 5,3 hat.

Beispiel 13: Apparatur gemäss Beispiel 11.
Nach dem Verfahren von Beispiel 10 werden 100 g einer 48,3%-igen Lösung (in Methoxypropylacetat) des Reaktionsproduktes gemäss Beispiel 5 (0,117 Mol Epoxygruppen), 8,85 g Acrylsäure (0,123 Mol), 0,15 g Hydrochinonmonomethylether und 1,15 ml einer 10%-igen Lösung (in Methoxypropylacetat) von Nuosynchromium 5 umgesetzt. Die Reaktion ist nach 7 Stunden bei 110°C Innentemperatur beendet, die Säuretitration ergibt dann 0,06 Mol/kg.
Analytische Daten des Reaktionsproduktes (210 g):

| | |
|---|---|
| Epoxywert (titrimetrisch) | 0,020 Mol/kg. |

Das Produkt entspricht der Formel gemäss Beispiel 12.

Beispiel 14: Apparatur (1000 ml Reaktor) gemäss Beispiel 10.
Nach dem Verfahren von Beispiel 10 werden 720 g einer 50%-igen Lösung (in Methoxypropylacetat) des Reaktionsproduktes gemäss Beispiel 5 (0,828 Mol Epoxygruppen); 29,83 g Acrylsäure (0,414 Mol), 1,11 g Hydrochinonmonomethylether und 37,00 ml einer 2%-igen Lösung (in Methoxypropylacetat) von Nuosynchromium 5 umgesetzt. Nach 5 Stunden bei 100°C Innentemperatur ist die Reaktion beendet, die Säuretitration ergibt dann <0,02 Mol Säure/kg.
Analytische Daten dieses Reaktionsproduktes:

| | | |
|---|---|---|
| 1. | Festkörpergehalt | 50,3%; |
| | | |
| 2. | Epoxywert (titrimetrisch) | 0,65 Mol/kg ; |
| | | |
| 3. | GPC (Polystyrol-Eichung) | M_{w} = 38840; Mₙ = 4226. |

Der Festkörper entspricht der Formel III, worin A und T die im Beispiel 1 und Q, L und M die im Beispiel 11 angegebene Bedeutung haben, und n einen Wert von etwa 5,3 hat.

### c) Carboxylgruppenhaltige Epoxyacrylate der vollständig acrylierten PGEH

Beispiel 15: Als Apparatur verwendet man ein 5000 ml Reaktionsgefäss, ausgestattet mit Rührer, Thermometer, Rückflusskühler und einem Einleitungsrohr für Luft. Zur Inhibierung der Polymerisation der Acrylate wird während der Reaktion ein schwacher Luftstrom unter Niveau eingeleitet. Die Heizung geschieht mittels thermostatisierbarem Oelbad. Im Reaktor werden 2171 g des Reaktionsproduktes gemäss Beispiel 9 (2,61 Mol OH-Gruppen) vorgelegt und unter Rühren 196,08 g (1,96 Mol) Bernsteinsäureanhydrid zugegeben. Man gibt 7,4 ml Benzyldimethylamin zu, erwärmt unter Einleiten eines trockenen Luftstroms auf 110°C Innentemperatur und lässt während 4 Stunden bei dieser Temperatur reagieren. Dann werden nochmals 7,4 ml Benzyldimethylamin zugefügt und weitere 4 Stunden bei dieser Temperatur weitergerührt. Das homogene, leicht gelbliche Reaktionsprodukt wird abgekühlt und ohne zusätzliche Reinigung weiterverwendet.
Analytische Daten dieses Reaktionsproduktes:

| | | |
|---|---|---|
| 1. | Festkörpergehalt | 55%; |
| | | |
| 2. | Säuregehalt (titrimetrisch) | 0,88 Mol/kg ; |
| | | |
| 3. | GPC (Polystyrol-Eichung) | M_{w} = 8525; Mₙ = 2237. |

Der Festkörper entspricht der Formel IV, worin A und T die im Beispiel 1 angegebene Bedeutung haben, und
- X: 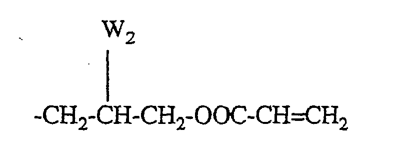
- Y: 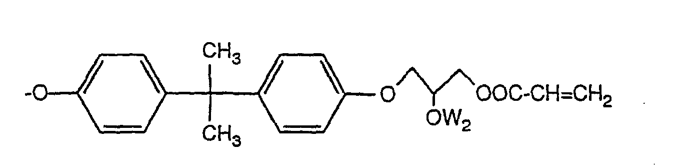
- W₂: -H oder 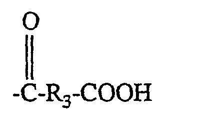
- W₁: -H oder 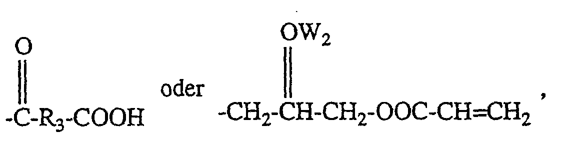
- R₃: -CH₂-CH₂- und
- n: 2,0 bedeuten.

Beispiel 16: Apparatur (500 ml) gemäss Beispiel 15.
Im Reaktor werden das Reaktionsprodukt aus Beispiel 13 (0,117 Mol OH-Gruppen) und 5,854 g Bernsteinsäureanhydrid (0,0585 Mol) vorgelegt und auf 110°C aufgeheizt. Unter Einleiten eines schwachen Luftstroms lässt man 7 Stunden bei dieser Temperatur reagieren. Das homogene Reaktionsprodukt wird ohne zusätzliche Reinigung weiterverwendet.

Analytische Daten dieses Reaktionsproduktes:

| | | |
|---|---|---|
| 1. | Festkörpergehalt | 61,7%; |
| | | |
| 2. | Säuregehalt (titrimetrisch) | 0,66 Mol/kg . |

Der Festkörper entspricht der Struktur gemäss Beispiel 15 mit einem n Wert von ungefähr 5,3.

### Applikationsbeispiele

Generelles: Als Beschichtungssubstrate dienen gereinigte kupferkaschierte Elektroniklaminate oder prozessierte Leiterplatten, die ein Leiterbahnmuster aufweisen.
Die Resistrezepturen werden durch Zusammenmischen und Lösen der in den Beispielen aufgeführten Komponenten, eventuell gefolgt von einer Filtration, hergestellt.
Alle Operationen sind unter Gelblichtschutz durchzuführen.
Für Testzwecke kann man die Rezepturen mit einem Drahtrakel auf die Leiterplatte beschichten. Für grössere Serien werden vor allem Vorhanggiessverfahren oder Walzenbeschichtung sowie Siebdruckverfahren eingesetzt.
Die Trocknung geschieht in einem Umluftofen. Zur Belichtung verwendet man kommerzielle Geräte mit 5000 W Metallhalogenid - dotierten Quecksilberhochdruckstrahler. Die Entwicklung wird in kommerziellen Durchlaufentwicklungsgeräten durchgeführt Zur Beurteilung der Photoempfindlichkeit und des Auflösungsvermögens belichtet man durch Stufenkeil und Auflösungskeil der Fa. Stouffer und evaluiert das Ergebnis anhand des entwickelten Resistbildes.

### Applikationsbeispiel

Rezeptur 1.1 enthält ein erfindungsgemässes carboxylgruppenmodifiziertes Acrylat:

| | |
|---|---|
| 53,00 g | Reaktionsprodukt gemäss Herstellungsbeispiel 15 (als 55%-ige Lösung in Methoxypropylacetat); |
| 19,00 g | CN 965 (Acrylat der Fa. Craynor); |
| 3,00 g | Irgacure 907 (Photoinitiator der Fa. CIBA AG, Basel); |
| 1,50 g | Quantacure ITX (Isopropylthioxanthon; Sensibilisator); |
| 0,15 g | Orasolblau GN (Farbstoff der Fa. CIBA AG, Basel); |
| 28,95 g | Methoxypropylacetat. |

Rezeptur 1.2: Vergleichsbeispiel, mit einem hochmolekularen Binder ohne erfindungsgemässe Acrylate:

| | |
|---|---|
| 97,20 g | Scripset 550E-Lösung (30%-ige Lösung in Methoxypropylacetat; Bindemittelpolymer der Fa. Monsanto; Styrolmaleinsäurecopolymer); |
| 19,00 g | CN 965 ; |
| 3.00 g | Irgacure 907; |
| 1,50 g | Quantacure ITX; |
| 0,15 g | Orasolblau GN. |

### Ergebnis:

| | Rezeptur 1.1 | Rezeptur 1.2 |
|---|---|---|
| Festkörpergehalt | 50% | 43,7% |
| Viskosität 25°C (Epprecht) | 600 mPa.s | 7600 mPa.s |
| Trockenschichtdicke | 12µm | 12µm |
| Trocknungsbedingungnen | 5 Min. 80°C | 5 Min. 80°C |
| Belichtung | 150 mJ/cm² | 150 mJ/cm² |
| Entwicklung | 1% Natriumcarbonat 35°C | 1% Natriumcarbonat 35°C |
| Letzte sichtbare Keilstufe | 6 | kein Bild: wegentwickelt |

Die Rezeptur 1.1 mit dem erfindungsgemässen Acrylat weist trotz dem wesentlich höheren Festkörpergehalt eine bedeutend niedrigere Viskosität als diejenige der Rezeptur 1.2 auf. Zudem ist die Photoempfindlichkeit (Stouffer Keilstufe 6) im Vergleich zur Rezeptur 1.2 viel höher (zu geringe Empfindlichkeit, um ein Bild zu erzeugen).

## Patentansprüche

1. Carboxylgruppenhaltige Epoxyacrylate der Formel IV 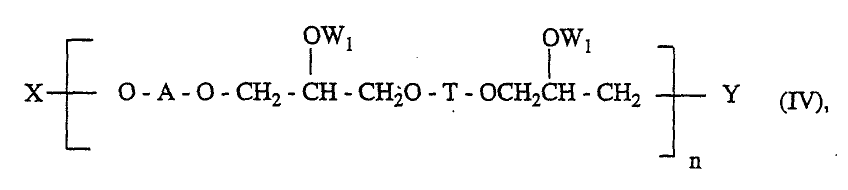 worin
x Wasserstoff oder eine Gruppe der Formeln 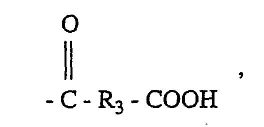 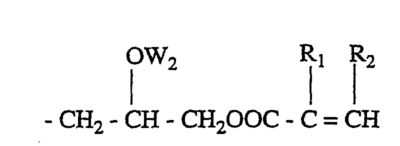 oder 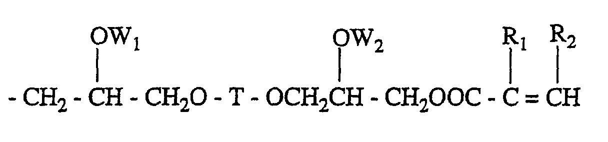 bedeutet,
R₃ den Rest des Bernsteinsäureanhydrid nach Entfernung des Anhydridrestes,
W₁ Wasserstoff oder eine Gruppe der Formeln 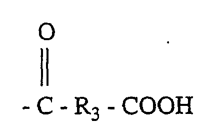 oder 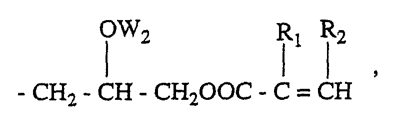
W₂ -H oder die Gruppe 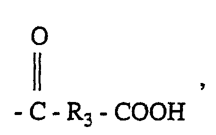 und
Y die Gruppe der Formeln -O-A-O-W₁ oder 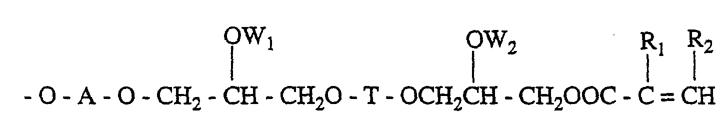 bedeuten, und
worin
A und T den Rest einer bifunktionellen aromatischen Verbindung,
R₁ -H oder -CH₃,
R₂ -H, -CH₃ oder Phenyl, und
n eine ganze Zahl von 0 bis 300 bedeuten,
wobei in der Formel IV mindestens 10 Mol % der Reste W₁, die nicht in den Endgruppen X und Y sind, eine Gruppe der Formel 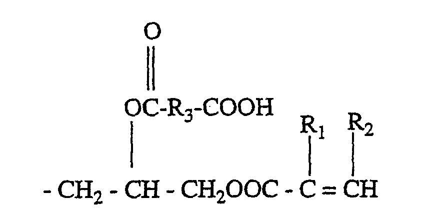 darstellen.

2. Verfahren zur Herstellung der carboxylgruppenhaltigen Epoxyacrylate der Formel IV gemäss Anspruch 1, **dadurch gekennzeichnet, dass** ein Epoxyacrylat der Formel III 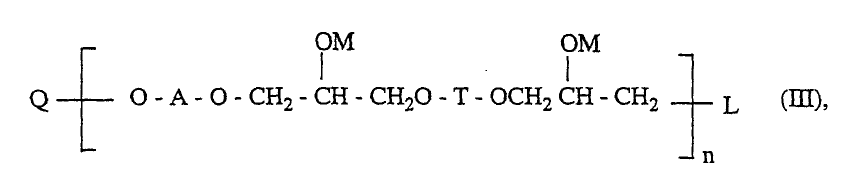 worin
Q Wasserstoff oder eine Gruppe der Formeln 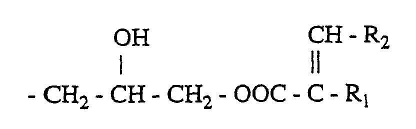 oder 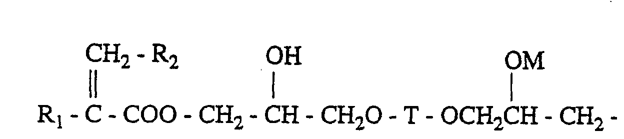 bedeutet,
R₁ -H oder -CH₃,
R₂ -H, -CH₃ oder Phenyl,
T den Rest einer bifunktionellen aromatischen Verbindung,
M Wasserstoff oder eine Gruppe der Formel 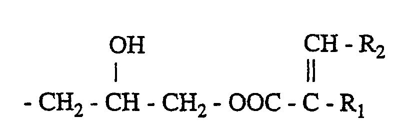 bedeuten, wobei
R₁ und R₂ die oben angegebene Bedeutung haben,
A den Rest einer bifunktionellen aromatischen Verbindung,
n eine ganze Zahl von 0 bis 300, und
L eine Gruppe der Formeln 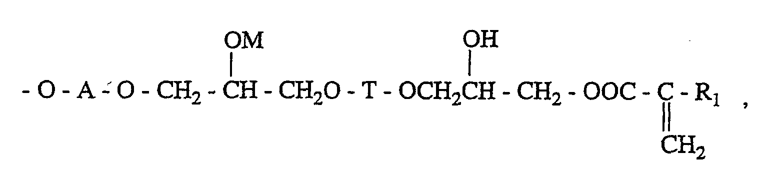 oder - O - A - OM bedeuten,
wobei in der Formel III nicht alle Reste M gleichzeitig Wasserstoff bedeuten können, sondern mindestens 10 Mol %, vorzugsweise 20-100 Mol % der Reste M, die nicht in den Endgruppen Q und L sind, eine Gruppe der Formel 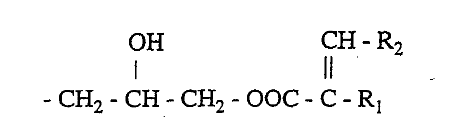 darstellen, wobei R₁ und R₂ die im Anspruch 1 angegebene Bedeutung haben, mit Bernsteinsäureanhydrid gegebenenfalls in Anwesenheit eines Katalysators und eines Polymerisationsinhibitors bei erhöhter Temperatur umgesetzt wird.

3. Verfahren gemäss Anspruch 2, worin in der Formel III R₁ Wasserstoff oder Methyl und R₂ Wasserstoff, Methyl oder Phenyl bedeuten.

4. Verfahren gemäss Anspruch 2, worin in der Formel III n eine ganze Zahl von 0 bis 50 und A und T unabhängig voneinander ein Brückenglied der Formel 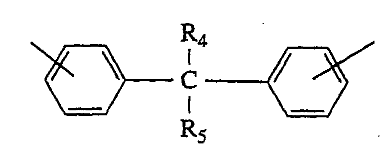 bedeuten,
wobei R₄ und R₅ unabhängig voneinander -H oder C₁-C₄-Alkyl sind und die Phenylreste des Brückengliedes unsubstituiert oder durch Brom substituiert sind.

5. Verwendung der carboxylgruppen enthaltenden Epoxyacrylate der Formel IV gemäss Anspruch 1 als Acrylatkomponenten in Photoresistformulierungen.

## Claims

1. A carboxyl group-containing epoxy acrylate of formula IV 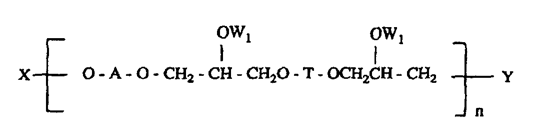 wherein
x is hydrogen or a group of formula 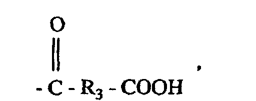 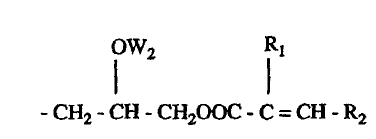 or 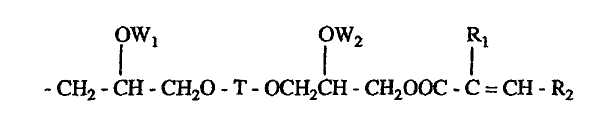
R₃ is the radical of succinic anhydride of a polycarboxylic acid after removal of the anhydride radical,
W₁ is hydrogen or a group of formula 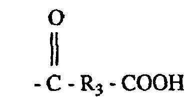 or 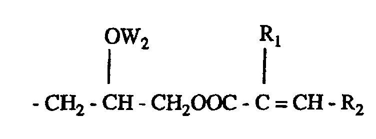
W2 is -H or the group 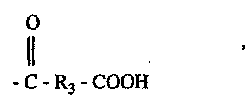 and
Y is the group of formula -O-A-O-W₁ or 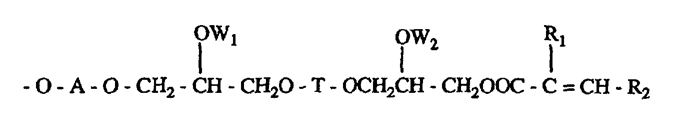
wherein
A and T are the radical of an aromatic bifunctional compound,
R₁ is -H or -CH₃,
R₂ is -H or -CH₃ or phenyl, and
n is an integer from 0 to 300,
with the proviso that, in formula IV, at least 10 mol % of radicals W₁ that are not in the end groups X and Y are a group of formula

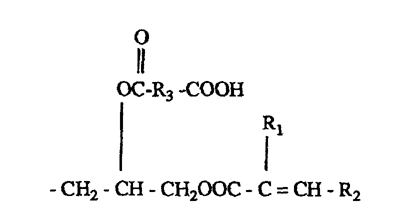

2. A process for the manufacture of carboxylic groups containing expoxy acrylates of formula IV according to claim 1, **characterized in that** 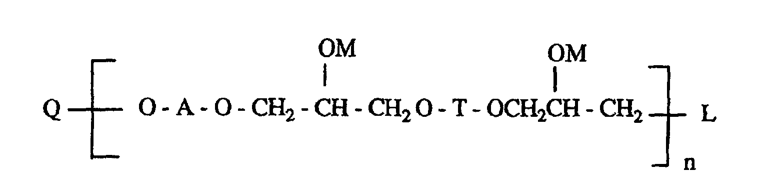 wherein
Q is hydrogen or a group of formula 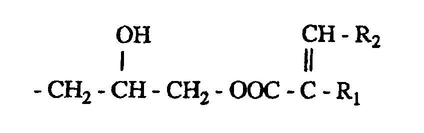 or 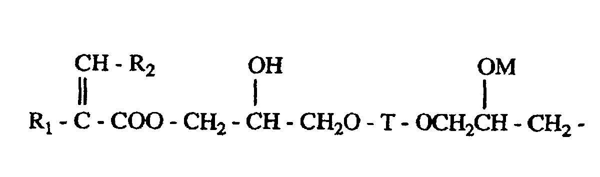
R₁ is -H or -CH₃,
R₂ is -H, -CH₃ or phenyl
T is the radical of an aromatic bifunctional compound, and
M is each independently hydrogen or a group of formula 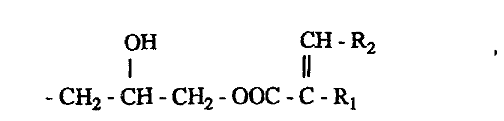 in which
R₁ and R₂ are as defined above,
A is the radical of an aromatic bifunctional compound,
n is an integer from 0 to 300, and
L is a group of formula 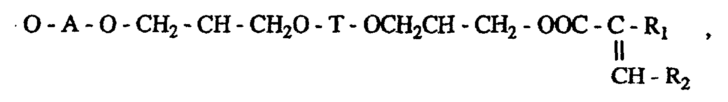 or
-O-A-OM ,
with the proviso that in formula III not all radicals M may be simultaneously hydrogen, but at least 10 mol %, preferably 20-100 mol %, of the radicals M that are not present in the end groups Q and L denote a group of the above formula 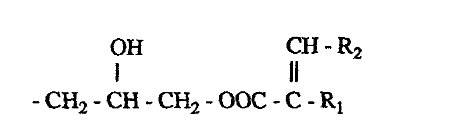 wherein R₁ and R₂ have the meanings given in claim 1, are reacted with succinic acid anhydride optionally in presence of a catalyst and a polymerisation inhibitor at elevated temperatures.

3. A process according to claim 1, wherein R₁ is hydrogen or methyl and R₂ is hydrogen, methyl or phenyl.

4. A process according to claim 1, wherein n is an integer from 0 to 50 and A and T are each independently of the other a linking group of formula 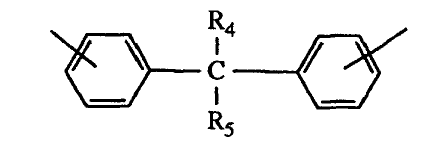 wherein R₄ and R₅ are each independently of the other -H or C₁-C₄ alkyl and the phenyl radicals of said linking group are unsubstituted or bromine-substituted.

5. Use of a carboxyl group-containing epoxy acrylate of formula IV as claimed in claim 1 as acrylate component in photoresist formulations.

## Revendications

1. Epoxyacrylates contenant des groupes carboxyle, de formule IV 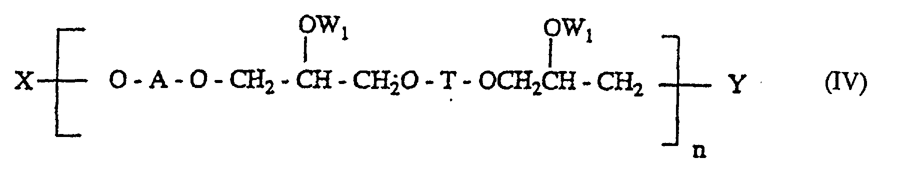 dans laquelle
X représente un atome d'hydrogène ou un groupe de formule 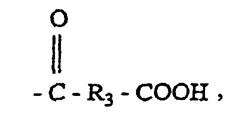 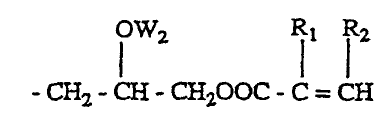 ou 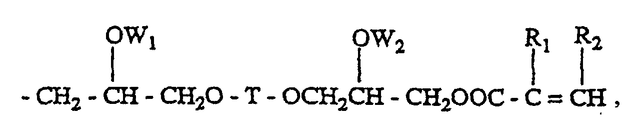 R₃ représente le reste de l'anhydride succinique après séparation du reste anhydride,
W₁ représente un atome d'hydrogène ou un groupe de formule 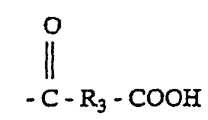 ou 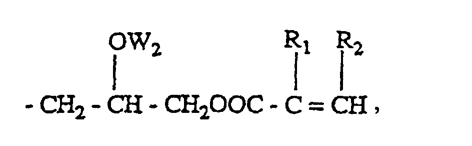 W₂ représente -H ou le groupe 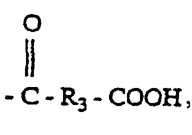 et
Y représente le groupe de formule -O-A-O-W₁ ou 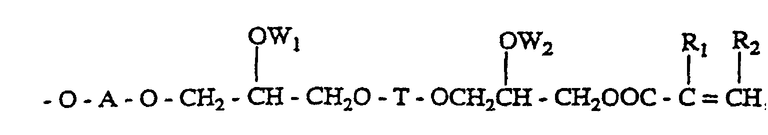 et où
A et T représentent le reste d'un composé aromatique bifonctionnel,
R₁ représente -H ou le groupe -CH₃,
R₂ représente -H ou un groupe -CH₃ ou phényle, et
n est un nombre entier de 0 à 300,
et, dans la formule (IV), au moins 10 % des restes W₁ qui ne se trouvent pas dans les groupes terminaux X et Y représentent un groupe de formule

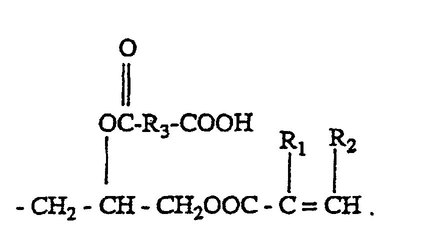

2. Procédé de préparation des époxyacrylates contenant des groupes carboxyle de formule IV selon la revendication 1, **caractérisé en ce que** l'on fait réagir un époxyacrylate de formule III 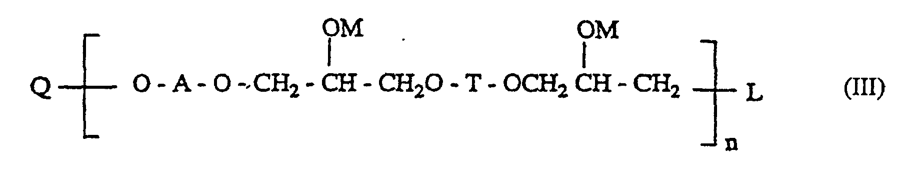 dans laquelle
Q représente un atome d'hydrogène ou un groupe de formule 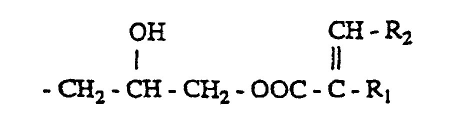 ou 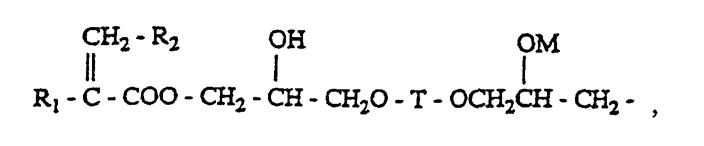 R₁ représente -H ou le groupe -CH₃,
R₂ représente -H ou un groupe -CH₃ ou phényle,
T représente le reste d'un composé aromatique bifonctionnel,
M représente un atome d'hydrogène ou un groupe de formule 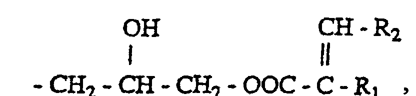 où R₁ et R₂ ont la signification indiquée ci-dessus,
A représente le reste d'un composé aromatique bifonctionnel,
n est un nombre entier de 0 à 300, et
L représente un groupe de formule 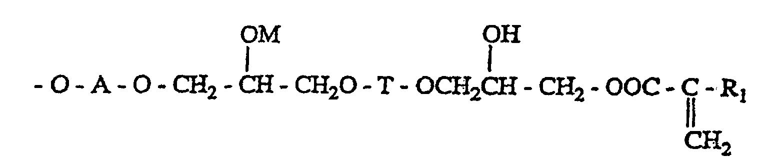 ou
-O-A-OM,
et, dans la formule III, tous les restes M ne peuvent pas être en même temps des atomes d'hydrogène, mais au moins 10 % en mol, de préférence 20 à 100 % en mol des restes M qui ne se trouvent pas dans les restes terminaux Q et L représentent un groupe de formule 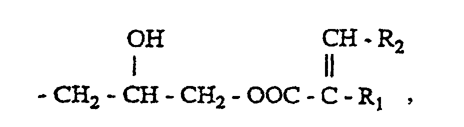 dans laquelle R₁ et R₂ ont la signification donnée dans la revendication 1,
avec de l'anhydride succinique, éventuellement en présence d'un catalyseur et d'un inhibiteur de polymérisation à température élevée.

3. Procédé selon la revendication 2, dans lequel, dans la formule III, R₁ représente un atome d'hydrogène ou un groupe méthyle et R₂ représente un atome d'hydrogène ou un groupe méthyle ou phényle.

4. Procédé selon la revendication 2, dans lequel, dans la formule III, n représente un nombre entier de 0 à 50 et A et T représentent, indépendamment l'un de l'autre, un élément de pontage de formule 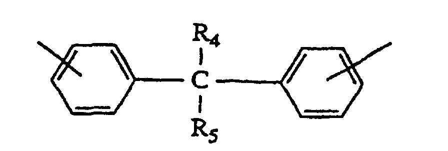 dans laquelle R₄ et R₅ représentent, indépendamment l'un de l'autre, un atome d'hydrogène ou un groupe alkyle en C₁-C₄ et les restes phényle de l'élément de pontage sont non substitués ou substitués par du brome.

5. Utilisation des époxyacrylates contenant des groupes carboxyle de formule IV selon la revendication 1 comme constituants acrylate dans des formulations de photorésists.
